# EUROPEAN PATENT APPLICATION

(11) **EP 1 705 171 A1**
(43) Date of publication of application: **27.09.2006**
(21) Application number: 06076289.5
(22) Date of filing: 12.07.2002
(51) Int. Cl.: C07C 47/225, A61Q 13/00, A61Q 15/00, A61K 8/49, A61K 8/33, C11D 3/50, A23L 1/226, C07C 47/293, C07C 47/395, C07D 333/22

(54) **Improved aromachemicals**

(30) Priority: 19.12.2001 US 342150 P; 15.01.2002 US 348580 P; 03.05.2002 US 377914 P; 17.06.2002 US 389298 P; 02.07.2002 US 355052 P
(62) Divisional of application: 02752283.8
(71) Applicant: Flexitral, Inc., Chantilly, VA 20151 (US)
(72) Inventor: Turin, Luca, London NW1 7NB (GB)
(74) Representative: Crowhurst, Charlotte Waveney

(57) **Abstract**

The present invention provides a derivative of an aromachemical obtainable by a process which comprises replacing at least one double bond in an aromachemical by a three-membered ring, wherein the three membered ring includes the two carbons of the double bond, each of which is bonded to an oxygen, a sulfur or a C(R)₂ group, wherein R is, independently, H, C₁₋₅ alkyl or C₁₋₅ substituted alkyl, and wherein the substituents on the substituted alkyl groups are selected from the group consisting of halo, hydroxy, thiol, thioether, amine, carboxylic acid, ester, nitro, cyano, sulfonic acid, urea, and thiourea.

## Description

### FIELD OF THE INVENTION

The present invention relates generally to the field of flavorings and fragrances. More particularly, the present invention relates to perfumes and other fragrant articles based on aromachemicals which overcome the stability limitations and/or allergic nature of the native compounds.

### BACKGROUND OF THE INVENTION

Many aromachemicals are used in the flavoring and fragrance industries. For example, citral has a lemon scent and as such is used as a flavor and/or fragrance in many articles of manufacture. However, many aromachemicals include double bonds, aldehyde groups and other reactive groups which are potentially susceptible to reaction and may result in a limited useful lifetime. Further, many essential oil fragrances have recently been determined to cause allergic reactions, and it is becoming increasingly difficult to bring such compounds to market.

Many aromachemicals, which are fundamental to the formation of various fragrances have been placed on the allergens list and are being banned or restricted in many commercial regions. The bans or restrictions will undoubtedly have a considerable effect on the quality of various fragrances, largely because the reduction in the perfumers pabtte makes the creation of certain notes virtually impossible. Examples of aromachemicals used to form muguet accords include hydroxycitronellal, lyral (IFF) lilial, and bourgeonal. The only lily-of-the-valley materials left to the perfumer are the cruder odorants like cyclamen aldehyde, majantol (Haarman &Reimer), mayol (Firmenich), and the newer dupical (Quest), elintaal (Quest), florahydral (Givaudan-Roure). Even rose notes will become difficult to create with both geraniol and citronellol on the list, and many other classical and fantasy odour notes and themes will become difficult to achieve, with the perfumer being effectively hand-cuffed.

It would be desirable to develop derivatives of these compounds that do not similarly result in allergic reactions and/or which have improved useful lifetimes. Additionally beneficial properties include improved odor intensity and stability. The present invention provides such fragrances and flavorings.

### SUMMARY OF THE INVENTION

Improved fragrances and flavorings that have a longer useful shelf life than the parent compounds from which they are derived are disclosed. In particular, derivatives of aromachemicals that maintain the fragrance characteristics of the aromachemicals, while lowering the allergic properties, and which can possess a longer shelf-life than the parent compounds from which they are derived, are disclosed. Also disclosed are methods of making the derivatives, and articles of manufacture including the derivatives.

In one embodiment, the parent compounds include one or more double bonds and the derivatives are prepared by replacing one or more double bonds in the parent molecule with a thioether linkage, cyclopropyl group, oxirane group, or thiirane group, where the cyclopropyl group can be unsubstituted, or substituted with one or two lower alkyl, preferably methyl groups. The alkyl groups can optionally be substituted, for example, with electron donating groups, electron with drawing groups, groups which increase the hydrophilicity or hydrophobocity, and the like.

Where these parent compounds further include one or more aldehyde groups, derivatives can be prepared where at least one aldehyde group in the parent molecule is replaced with a nitrile, methyl ether or acetal group. The acetal groups can provide the compounds with a long lasting flavor or fragrance, where the acetals slowly hydrolyze to provide the parent aldehyde compounds. In some embodiments, suitable molecules include both aldehyde and double bond functional groups, which are both derivatized as described herein.

In a third embodiment, the parent compounds include a benzene ring, and the derivatives are prepared by replacing the benzene ring with a thiophene ring. The thiophene may include one or more C1-5 alkyl groups, preferably in the 2 and/or 3-position. Some known compounds exist that have similar odor character where a phenyl group is present in one molecule and an isoprenyl group in the other. In a fourth embodiment of the present invention, the parent compounds include an isobutenyl group or a phenyl group, and the derivatives are prepared by replacing the isobutenyl or phenyl group with a cyclopropanated isoprenyl group.

Examples of suitable articles of manufacture include candles, air fresheners, perfumes, disinfectant compositions, hypochlorite (bleach) compositions, beverages such as beer and soda, denture cleanser tablets as described, for example, in U.S. Patent No. 5,571,519, the contents of which are hereby incorporated by reference in their entirety, and flavored orally-delivered products such as lozenges, candies, and the like.

### BRIEF DESCRIPTION OF THE DRAWINGS

Figures 1a-d are vibrational spectra of a cyclopropyl derivative of rose oxide (la), linalool (1b), limonene (1c) and ionone (1d).
Figure 2 is a vibrational spectra of a cyclopropyl derivative of lyral.
Figures 3a and 3b are vibrational spectra for cis and trans-4-methyl-2-phenyl-2-pentenal, respectively.
Figure 4 is a representative list of aromachemicals that can be modified using the chemistry described herein.

### DETAILED DESCRIPTION OF THE INVENTION

Improved fragrances and flavorings that have a longer useful shelf life than the parent compounds from which they are derived are disclosed. The improvements can be in the form of greater intensity and/or greater chemical stability without change in odor character. If greater intensity is desired, then the odorant structure is modified in order to increase the intensity of the odor, such as by increasing zinc-binding ability, without significantly changing odor character. If greater stability is desired, then one or more structural features responsible for chemical instability can be altered as described herein without significantly changing odor character.

### I. Isodonic molecules

The derivatives described herein are isodonic to the compounds from which they can be derived. By isodonic is meant "having essentially the same odor profile." However, while the compounds may have essentially the same odor profile, they have improved stability, odor intensity and/or other improved physical and/or chemical properties.

The compounds from which the derivatives can be derived are aromachemicals, for example, specific odorant compounds present in essential oils. The derivatives can be prepared from the aromachemicals or the individual compounds, but need not be. That is, the compounds can be derived from synthetic strategies that do not involve using the aromachemicals, so long as the ultimate compound is a derivative of the oils or specific odorant compounds as described herein. All that is required is that the compounds are isodonic with the "parent" compounds. Isodonic replacements (for example, ene-cyclopropane replacement, ene-oxirane replacement, ene-thiirane replacement, ene-thioether replacement, isobutenyl-phenyl replacement and benzene-thiophene replacement) are described in more detail below. In some embodiments, the parent compounds include an aldehyde group, nitrile group, methyl ether group and/or ester group in addition to the olefinic, phenyl and/or thiophene group. In these embodiments, in addition to the replacements described above, the following additional replacements can further be made: aldehyde-nitrile replacement, aldehyde-methyl ether replacement, aldehyde-acetal replacement, aldehyde-ester replacement, and inverses of these replacements (i.e., methyl ether-aldehyde and the like).

The odorant intensity and/or stability of aromachemicals can be improved by replacing a common chemical feature with another designed to alter the chemistry while leaving the basic structure, and therefore the odor itself, virtually untouched. Examples of suitable chemical features that can be replaced are described in more detail below.

### Double-bond Replacements and their Effect on Odor

Many aromachemicals include an isoprenyl unit and/or other C=C double bonds. The C=C double bonds can be replaced by thioether groups, -S-, without marked change in odor character. In one embodiment, the carbon double bond is replaced with a thiocyclopropane ring. In this embodiment, the lone pair of electrons on sulfur binds readily to Zn, which increases the odor intensity without significantly altering the odor type.

One or more (if present) C=C double bonds in an essential oil can be replaced with cyclopropane, oxirane (OX), or thiirane (TH) moieties. The odor of the compounds remains substantially the same with this substitution; whereas the odorant intensity can be dramatically improved. One way to measure the odorant intensity is through zinc binding affinity. All three 3-membered rings described above can coordinate to a zinc ion.

Another advantage of replacing the C=C double bond with an oxirane or thiirane is that this produces a molecule with a higher molecular weight. The greater molecular weight can lower the volatility of the molecule, thereby potentially changing a top note to a middle note, or a middle note to a drydown note.

The procedure described herein for improving the performance of an odorant is best illustrated with citral. It is immediately applicable to any other odorants possessing the same structural features, namely a C=C double bond. Citral can be cyclopropanated in one or both of the positions, corresponding to the two double bonds of the molecule. Including stereoisomers, there are thus five CP-variations on citral, shown below.

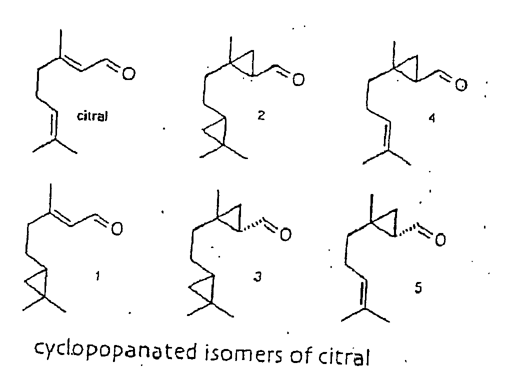

The cyclopropane rings can include a CH₂ moiety, or can be substituted with one or two methyl groups. The methyl or dimethyl analogues have a vibrational spectra that more closely matches citral than the unsubstituted cyclopropane derivatives, and has a sweeter smell than the unsubstituted cyclopropyl derivatives.

The derivatives described herein include derivatives in which one or both of the double bonds is replaced with a (unsubstituted, monoalkyl or dialkyl, where alkyl can be substituted or unsubstituted, and is preferably methyl) cyclopropyl group. The compounds can include, in combination with or in place of a cyclopropyl group, the replacement of the aldehyde with a methyl ether, a nitrile or an acetal group.

The synthesis of methyl, dimethyl or unsubstituted cyclopropane derivatives is well known to those of skill in the art, and involves, for example, bromoform reaction to form the dibromocyclopropane derivative, followed by stoichiometric reaction with methyl lithium. The aldehyde group is typically protected as an acetal during the reaction, and deprotected as desired after the reactions take place. In one embodiment, however, the acetals (for example, dimethyl, diethyl, or ethylene glycol) are not deprotected to the aldehyde, such that the flavoring or fragrance includes a portion or entirely the acetals. The acetals can then slowly hydrolyze over time, releasing the lemon scent/flavoring. Alternatively, derivatives including one or two cyclopropane rings can also include a nitrile or methyl ether group as a replacement for the aldehyde group.

These simple procedures yield derivatives with odor profiles close to the aromachemicals or individual "parent" compounds themselves. Further, by replacing the double bonds, the derivatives often have greater potency and far greater acid and bleach stability since the unstable feature, namely the double bond, has been removed.

The same applies to epoxide (OX), and thiirane (TH) rings. Not counting mixed C=C double bond replacements and stereoisomers, this generates 9 possible molecules from citral alone, all readily accessible in one or two step syntheses from citral by processes well known in the art, such as: .

**Cyclopropanyl replacement**: Simmons-Smith cyclopropanation of the aldehyde or corresponding alcohol, followed by periodinane oxidation for the latter to give the aldehyde¹
¹ Vogel's textbook of practical organic chemistry 5th edition (1989) pp 1106-1108

**Oxiranyl replacement**: m-chloroperbenzoic acid epoxidation²
² Ibid, pp1127-1129

Thiiranyl replacement: bromination of double bond on Amberlite, followed by S'-substitution in sodium sulfides³
³ Choi J et al (1995) Bull. Korean. Chern. Soc., 16, 189 - 190 Convenient Synthesis of Symmetrical Sulfides from Alkyl Halides and Epoxides

In another embodiment, the aromachemicals include an isobutenyl group, and the group is replaced with a phenyl group or vice versa.

### Aldehyde Replacement with Nitrile, Methyl Ester, or Acetal Groups

Many aromachemicals also include aldehyde groups. Odotopes of these aromachemicals can be prepared by replacing the aldehyde group with a nitrile, methyl ester or acetal group. The conversion of an aldehyde group to a nitrile group is well known in the art, and described, for example, in U.S. Patent No. 5,892,092. The '092 patent teaches a process for forming nitriles from aldehydes. Examples of oximes that can be formed from aldehydes and then converted to nitriles include angelica oxime, aleprine oxime, alpha,beta-apocitronellal oxime, bergamotene oxime, pyroterebine oxime, campholene oxime, citronellal oxime, citral oxime, chrysantheme oxime, cyclocitral oxime, cyclolavandulal oxime, faranal oxime, farnesal oxime, isolauranal oxime, ikema oxime, myrthenal oxime, phellandrene oxime, safranal oxime and sorbinal oxime. Specific examples include angelica oxime, bergamotene oxime, cyclolavandulal oxime, citral oxime, farnesal oxime, ikema oxime, isolauranal oxime, phellandrene oxime and sorbinal oxime.

Acetal formation is well known to those of skill in the art, and generally involves reacting an aldehyde with an alcohol in the presence of an acid catalyst. The acetal is formed with loss of water. In use, when the acetal is present in an aqueous environment, the acetal can revert to the aldehyde, thereby providing a time-release form of the odorant.

Aromachemicals including an aldehyde that further include one or more a double bonds can be converted to a thioether, (unsubstituted, methyl or dimethyl) cyclopropyl, oxirane, or thiirane derivative that also include a nitrile, methyl ether or acetal group in place of the aldehyde.

This method is immediately applicable to several other classes of odorants in order to increase their potencies. The following examples each denote a class of odorant, not a single molecule.

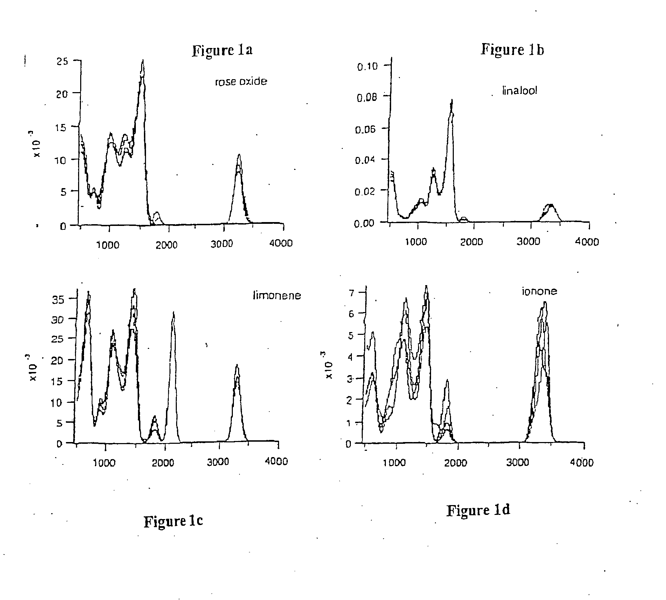

In each case the C=C double bond(s) can be substituted with CP, OX or TH to yield stronger odorants with similar odor profiles. Rose oxide is a floral, ionone a woodyviolets, damascone a fruity rose, sandanol a sandalwood, limonene a woody citrus, velvione a musk, linalool a floral-woody and ethyl citronellyl oxalate a musk. The graphs for four of these compounds with CP, OX and TH substitutions are shown in Figures 1a-d, where Figure 1a shows spectra of rose oxide, Figure 1b shows spectra for linalool, Figure 1c shows spectra for limonene and Figure Id shows spectra for ionone. In each case, the CP, OX and TH substitution has only a minor effect on spectrum and therefore on odor character.

### Benzene - Thiophene Replacement

An additional odotopic replacement is a benzene ring for a thiophene ring. For example, when the phenyl rings in lilial, cyclamenaldehyde and bourgeonal are replaced with thiophene, not only do the vibrational spectra overlap and the novel derivatives have the same odor characteristics, but also the intensity of the odor is enhanced. Each of these compounds further includes an aldehyde group that can additionally be replaced with nitrile, methyl ether or acetal functionality. Synthetic methods for replacing a phenyl ring in a molecule with a thiophene molecule are well known to those of skill in the art.

The parent compounds (lilial, cyclamenaldehyde and bourgeonal) and the' novel compounds including the thiophene ring substitution, are shown below: 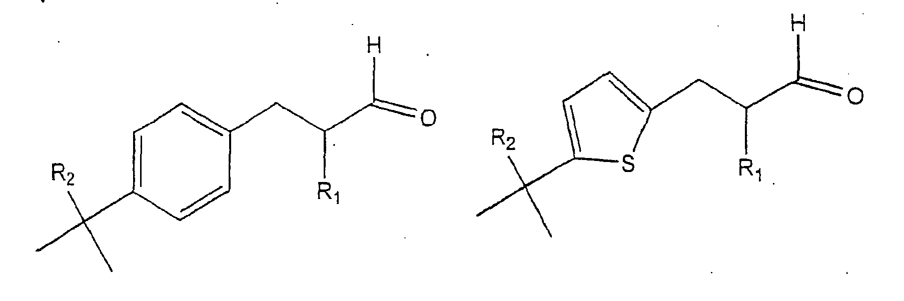 R1, R2=methyl: Lilial
R1 = methyl, R2 = H: cyclamenaldehyde
R1= H, R2 = methyl: Bourgeonal

An example of the benzene to cyclopropanated isobutenyl is the replacement of the phenyl group in 4-methyl-2-phenyl-pent-3-ene-1-al with a cyclopropanated isobutenyl moiety.

### II. Aromachemicals That Can be Modified Using the Chemistry Described Herein

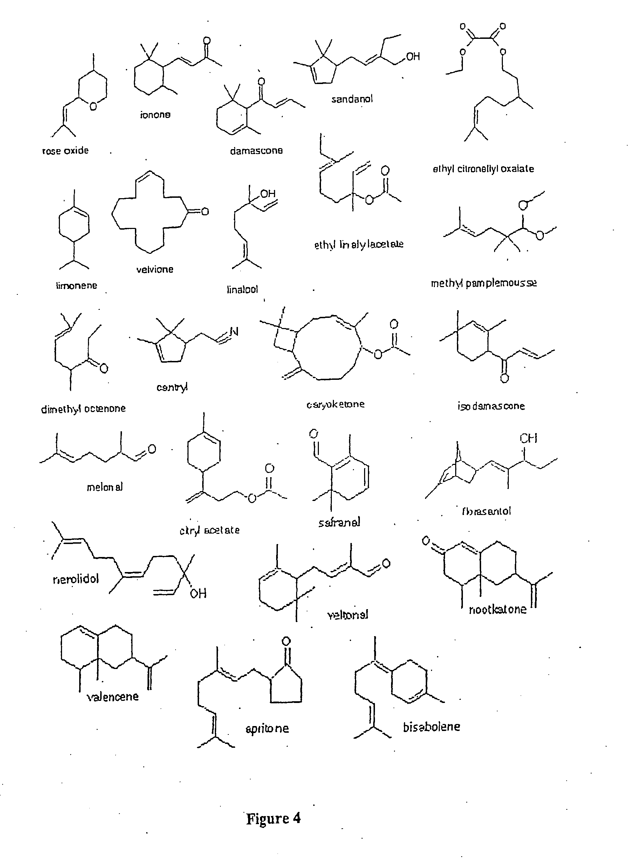

The technology described herein has particular application to aromachemicals, in particular, aromachemicals, including lyral, hydroxycitronellal and aldehydes and alcohols related to citral, including citronellal, geraniol, nerol and the like. Examples of aromachemicals that can be modified using the chemistry described herein are listed below, and also provided in Figure 4.

There are several aromachemicals with aldehyde groups that can be derivatized with acetal, methyl ether or nitrile groups using the chemistry described herein. These include, but are not limited to, angelica, aleprine, alpha,beta-apocitronellal, bergamotene, pyroterebine, campholene, citronellal, citral, chrysantheme, cyclocitral, cyclolavandulal, faranal, farnesal, isolauranal, ikema, myrthenal, phellandrene, safranal oxime and sorbinal oxime. Specific examples include angelica, bergamotene, cyclolavandulal, citral, famesal, ikema, isolauranal, phellandreneandrine oxime and sorbinal oxime.

There are several aromachemicals (several of which are listed above) that include olefinic groups that can be derivatized by forming an ene-thioether, ene-cyclopropane, ene-oxirane, and/or ene-thiirane replacement. The following are specific odorants that can be modified as described herein, wherein the double bonds can be converted to (unsubstituted, methyl or dimethyl) cyclopropyl derivatives, and/or the aldehydes (where present) converted to acetals, methyl ethers or nitriles without significantly altering the odor profile.

Amyl cinnamal (also known as 2-benzylidineheptanal and alpha-amyl cinnamic aldehyde)
Amyl cinnamyl alcohol (also known as 2-pentyl-3-phenylprop-2-ene-1-ol and alpha-amyl cinnamic alcohol)
cinnamyl alcohol (also known as cinnamic alcohol)
cinnamal (also known as3-phenyl-2-propenal and cinnamic aldehyde)
citral (also known as 3,7-dimethyl-2,6-octadiene-1-al, mix of cis and trans isomers)
coumarin (also known as 1-benzopyran-2-one or cis-o-coumarinic acid lactone)
eugenol
geraniol
hydroxycitronellal (also known as 7-hydroxycitronellal or laurine)
lyral (also known as hydroxymethyl-pentylcyclo-hexenecarboxaldehyde and 4,(4-hydroxy-4-methylpentyl)cyclohex-3-enecarbaldehyde
isoeugenol
benzoyl cinnamate (INCI), (also known as benzyl 3'-Phenyl-2-propenoate or cinnamein)
citronellol (also known as 3,7-dimethyl-6-octenol)
farnesol (also known as 3,7,11-trimethyldodeca-2,6,10-trienol
hexyl cinnamaldehyde (also known as alpha-hexyl cinnamaldehyde)
lilial (also known as lilestral; 2-(4-tert-butylbenzyl)proprionaldehyde, 4-(1,1-dimethylethyl)-alpha-methylbenzenepropanal,
p-tert-butyl-alpha-methylhydrocinnamaldehyde)
d-limonene (also known as (R)-p-mentha-1,8-diene
linalool,
damascones, and
gamma-methylionone ((also known as 3-methyl-4-(2,6,6-trimethyl-2-cyclohexen-1-yl)-3-butene-2-one.

Additionally, the compounds can be selected from anethole, anise oil, caraway oil, cardamom oil, carvone, coriander oil, eriodictyon, ethyl vanillin, fennel oil, glycyrrhiza, lavender oil, lemon oil, menthol, nutmeg oil, orange flower oil, peppermint, rosemary oil, rose oil, spearmint oil, thyme oil, tolu balsam and vanillin.

Additional examples include angelica, bergamotene, cyclolavandulal, citral, farnesal, ikema, isolauranal, phellandrene oxime and sorbinal oxime. In particular, citral oxime can be converted to geranonitrile.

In particular, lyral has the following formula, which includes a double bond:

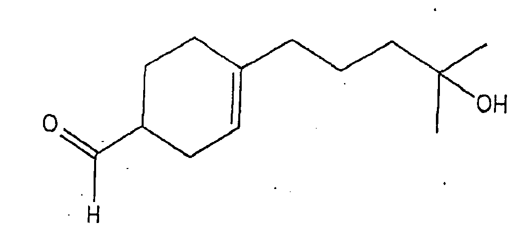

The cyclopropyl derivative of this compound was prepared (structure shown below):

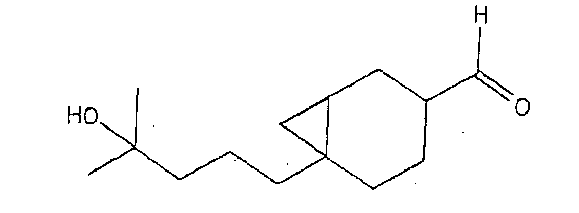

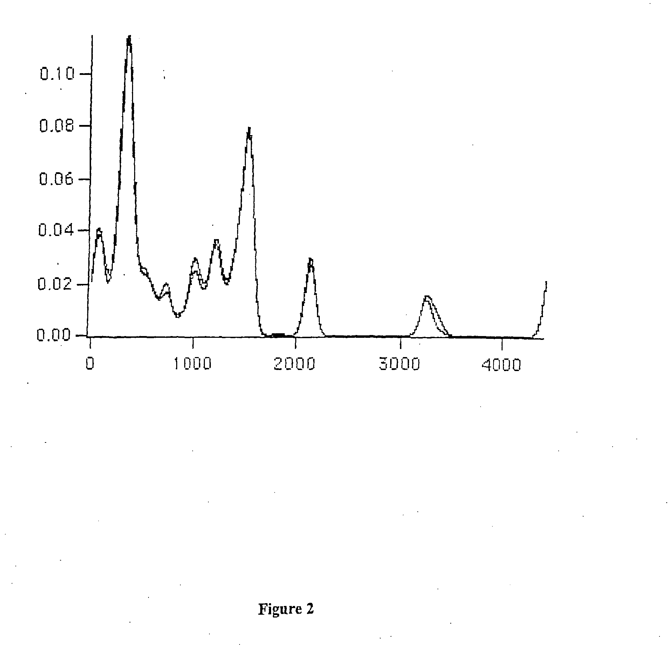

The vibrational spectra of the lyral and its cyclopropanated derivative substantially overlap, as shown in Figure 2. The fragrance of lyral and its cyclopropanated derivatives (for example, including one or two C₁₋₅ alkyl groups on the cyclopropane ring) substantially match. Suitable lyral odotopes include the unsubstituted, methyl and dimethyl cyclopropyl derivatives, as well as unsubstituted, methyl and dimethyl cyclopropyl derivatives where the aldehyde is replaced with a methyl ether, nitrile, or acetal.

Examples of parent ketones for these novel derivatives include alpha-ionone, beta-ionone, gamma-methyl ionone, irone alpha, methyl dihydrojasmonate, cis-jasmone, methyl amyl ketone, carvone, damascenone, alpha damascone, methyl beta-napthyl ketone, cassione, and menihone.

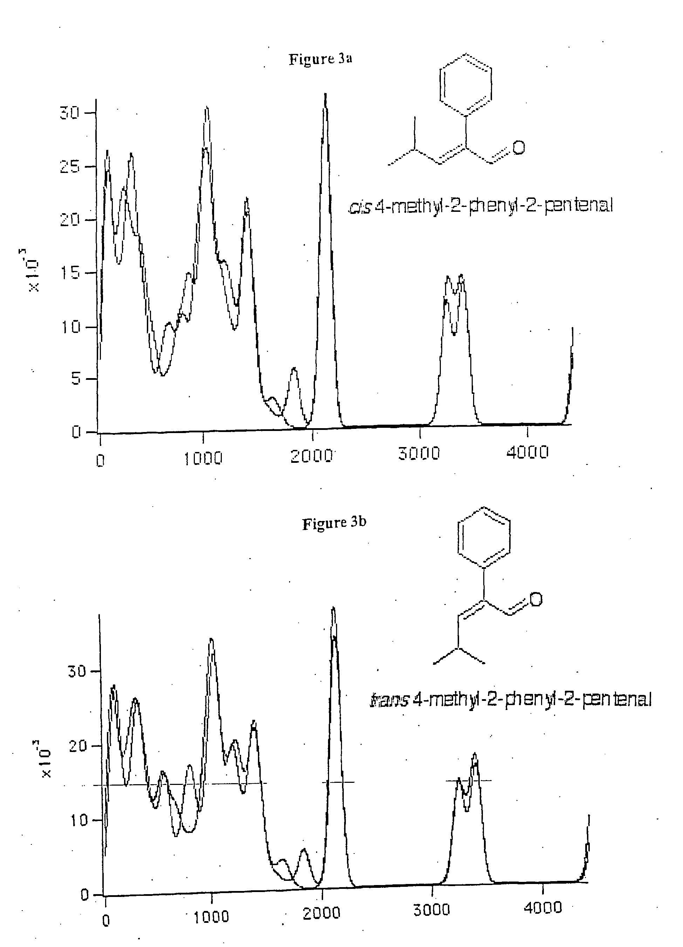

Still further examples include cis and trans-4-methyl-2-phenyl-2-pentenal. Replacement of the double bonds with cyclopropyl groups provide novel compounds with vibrational spectra that substantially overlap with the parent compounds. The spectra for cis and trans-4-methyl-2-phenyl-2-pentenal, respectively, are provided in Figures 3a and 3b.

### III. Articles of Manufacture Induding the Essential Oil Derivatives

The derivatives described herein can be included in virtually any article of manufacture that can include the aromachemicals or other "parent compounds" from which they are derived. Examples include bleach, detergents, flavorings and fragrances, beverages, including alcoholic beverages, and the like. The derivatives can be used in applications like soaps, shampoos, body deodorants and antiperspirants, solid or,liquid detergents for treating textiles, fabric softeners, detergent compositions and/or all-purpose cleaners for cleaning dishes or various surfaces, for both household and industrial use. Of course, the use of the compounds is not limited to the above-mentioned products, as they be used in other current uses in perfumery, namely the perfuming of soaps and shower gels, hygiene or hair-care products, as well as of body deodorants, air fresheners and cosmetic preparations, and even in fine perfumery, namely in perfumes and colognes. These uses are described in more detail below.

### Perfume Compositions

The compounds can be used as perfuming ingredients, as single compounds or as mixture thereof, preferably at a range of at least about 30% by weight of the perfume composition, more preferably at a range of at least about 60% by weight of the composition. The compounds can even be used in their pure state or as mixtures, without added components. The olfactive characteristics of the individual compounds are also present in mixtures thereof, and mixtures of these compounds can be used as perfuming ingredients. This may be particularly advantageous where separation and/or purification steps can be avoided by using compound mixtures.

In all cited applications, the derivatives can be used alone or in admixture with other perfuming ingredients, solvents or adjuvants of current use in the art. The nature and the variety of these co-ingredients do not require a more detailed description here, which, moreover, would not be exhaustive, and the person skilled in the art will be able to choose the latter through its general knowledge and as a function of the nature of the product to be perfumed and of the desired olfactive effect.

These perfuming ingredients typically belong to chemical classes as varied as alcohols, aldehydes, ketones, esters, ethers, acetates, nitrites, terpene hydrocarbons, sulfur- and nitrogen-containing heterocyclic compounds, as well as aromachemicals of natural or synthetic origin. A large number of these ingredients described in reference textbooks such as the book of S. Arctander, Perfume and Flavor Chemicals, 1969, Montclair, N.J., USA, the contents of which are hereby incorporated by reference in its entirety, or its more recent versions, or in other works of similar nature.

The proportions in which the derivatives can be incorporated in the various products vary within a large range of values. These values depend on the nature of the article or product that one desires to perfume and the odor effect searched for, as well as on the nature of the co-ingredients in a given composition when the compounds are used in admixture with perfuming co-ingredients, solvents or adjuvants of current use in the art.

As an example, the derivatives are typically present at concentrations between about 0.1 and about 10%, or even more, by weight of these compounds relative to the weight of the perfuming composition in which they are incorporated. Far lower concentrations than those mentioned above can be used when the compounds are directly applied for perfuming the various consumer products cited beforehand.

The compounds are relatively stable in typically aggressive media for perfumes. Accordingly, they can be used in detergents containing bleaching agents and activators such as, for example, tetraacetylethylenediamine (TAED), hypohalites, in particular hypochlorite, peroxygenated bleaching agents such as, for example, perborates, etc. The compounds can also be used in body deodorants and antiperspirants, for example, those containing aluminum salts. These embodiments are described in more detail below.

### Conventional Detergent Ingredients

In addition to the derivatives described herein, the compositions herein include a detersive surfactant and optionally, one or more additional detergent ingredients, including materials for assisting or enhancing cleaning performance, treatment of the substrate to be cleaned, or to modify the aesthetics of the detergent composition (e.g., perfumes, colorants, dyes, etc.). The following are illustrative examples of detersive surfactants and other detergent ingredients.

Detersive Surfactants Non-limiting examples of synthetic detersive surfactants useful herein typically at levels from about 0.5% to about 90%, by weight, include the conventional C₁₁₋₁₈ alkyl benzene sulfonates ("LAS") and primary, branch-chain and random C₁₀₋₂₀ alkyl sulfates ("AS"), the C₁₀₋₁₈ secondary (2,3) alkyl sulfates of the formula CH₃ (CH₂)ₓ (CH(CH₃)OSO₃⁻ M⁺) and CH₃(CH₂)y (CH(CH₂CH₂)OSO₃⁻ M⁺) wherein x and y are integers and wherein each ofx and (y+1) is least about 7, preferably at least about 9, and M is a water-solubilizing cation, especially sodium, unsaturated sulfates such as oleyl sulfate, the C₁₀₋₁₈ alkyl alkoxy sulfates ("AEx S"; especially EO 1-7 ethoxy sulfates), C₁₀₋₁₈ alkyl alkoxy carboxylates (especially the EO 1-5 ethoxycarboxylates), the C₁₀₋₁₈ glycerol ethers, the C₁₀₋₁₈ alkyl polyglycosides and their corresponding sulfated polyglycosides, and C₁₂₋₁₈ alpha-sulfonated fatty acid esters. If desired, the conventional nonionic and amphoteric surfactants such as the C₁₂₋₁₈ alkyl ethoxylates ("AE") including the so-called narrow peaked alkyl ethoxylates and C₆₋₁₂ alkyl phenol alkoxylates (especially ethoxylates and mixed ethoxy/propoxylates), C₁₂₋₁₈ betaines and sulfobetaines ("sultaines"), C₁₀₋₁₈ amine oxides, and the like, can also be included in the overall compositions. The C₁₀₋₁₈ N-alkyl polyhydroxy fatty acid amides can also be used. Typical examples include the C₁₂₋₁₈ N-methylglucamides. See WO 9,206,154. Other sugar-derived surfactants include the N-alkoxy polyhydroxy fatty acid amides, such as C₁₀₋₁₈ N-(3-methoxypropyl) glucamide. The N-propyl through N-hexyl C₁₂₋₁₈ glucamides can be used for low sudsing. C₁₀₋₂₀ conventional soaps may also be used, however synthetic detergents are preferred. If high sudsing is desired, the branched-chain C_{10 16} soaps may be used. Mixtures of anionic and nonionic surfactants are especially useful. Other conventional useful surfactants are listed in standard texts. See also U.S. Pat. No. 3,664,961, Norris, issued May 23, 1972.

Preferred compositions incorporating only synthetic detergents have a detergent level of from about 0.5% to 50%. Compositions containing soap preferably comprise from about 10% to about 90% soap.

Although the detergent compositions herein can consist of only detersive surfactant and pro-fragrance, the said compositions preferably contain other ingredients commonly used in detergent products.

### Builders

Detergent builders can optionally be included in the compositions herein to assist in controlling mineral hardness. Inorganic as well as organic builders can be used. Builders are typically used in fabric laundering compositions to assist in the removal of particulate soils.

The level of builder can vary widely depending upon the end use of the composition and its desired physical form. When present, the compositions will typically comprise at least about 1% builder. Liquid formulations typically comprise from about 5% to about 50%, more typically about 5% to about 30%, by weight, of detergent builder. Granular formulations typically comprise from about 10% to about 80%, more typically from about 15% to about 50% by weight, of the detergent builder. Lower or higher levels of builder, however, are not meant to be excluded.

Inorganic or detergent builders include, but are not limited to phosphate builders such as, the alkali metal, ammonium and allanolammonium salts of polyphosphates (exemplified by the tripolyphosphates, pyrophosphates, and glassy polymeric meta-phosphates), phosphonates, and phytic acid, and non-phosphorous builders such as silicates, carbonates (including bicarbonates and sesquicarbonates), sulphates, and aluminosilicates. Non-phosphate builders are required in some locales.

Organic builders suitable for use herein include polycarboxylate builders such as disclosed in U.S. Pat. No. 3,308,067, Diehl issued Mar. 7, 1967; U.S. Pat. No. 4,144,226, Crutchfield issued Mar. 13, 1979 and U.S. Pat, No. 4,246,495, Crutchfield, issued Mar. 27, 1979.

### Soil Release Agents

Soil Release agents are desirably used in laundry detergents of the instant invention. Suitable soil release agents include those of U.S. Pat. No. 4,968,451, Nov. 6, 1990 to J. J. Scheibel and E. P. Gosselink: such ester oligomers can be prepared by (a) ethoxylating allyl alcohol, (b) reacting the product of (a) with dimethyl terephthalate ("DMT") and 1,2-propylene glycol ("PG") in a two-stage transesterification/oligomerization procedure and (c) reacting the product of (b) with sodium metabisulfite in water; the nonionic end-capped 1,2-propylene/polyoxyethylene terephthalate polyesters of U.S. Pat. No. 4,711,730, Dec. 8, 1987 to Gosselink et al, for example those produced by transesterification/oligomerization of poly(ethyleneglycol) methyl ether, DMT, PG and poly(ethyleneglycol) ("PEG"); the partly- and fully-anionic-end-apped oligomeric esters of U.S. Pat. No. 4,721,580, Jan. 26, 1988 to Gosselink, such as oligomers from ethylene glycol ("EG"), PG, DMT and Na-3,6-dioxa-8-hydroxyoctanesulfonate; the nonionic-capped block polyester oligomeric compounds of U.S. Pat. No. 4,702,857, Oct. 27, 1987 to Gosselink, for example produced from DMT, Me-capped PEG and EG and/or PG, or a combination of DMT, EG and/or PG, Me-capped PEG and Na-dimethyl-5-sutfoisophthalate; and the anionic, especially sulfoaroyl, end-capped terephthalate esters of U.S. Pat. No. 4,877,896, Oct. 31, 1989 to Maldonado, Gosselink et al, the latter being typical of SRA's useful in both laundry and fabric conditioning products, an example being an ester composition made from m-sulfobenzoic acid monosodium salt, PG and DMT optionally but preferably further comprising added PEG, e.g., PEG 3400. Another preferred soil release agent is a sulfonated end-capped type described in U.S. Pat. No. 5,415,807.

### Other Optional Ingredients

The compositions herein can contain other ingredients such as enzymes, bleaches, fabric softening agents, dye transfer inhibitors, suds suppressors, and chelating agents, all well known within the art.

For purposes of defining detergent compositions of the present invention, the pH of the detergent composition is that which is measured at 1% concentration of the detergent composition in distilled-water at 20C. The detergent compositions herein have a pH of from about 7.1 to about 13, more typically from about 7.5 to about 9.5 for liquid detergents and from about 8 to about 12 for granular detergents.

### Formulation with Detergents With or Without Conventional Perfumery Materials

While the derivatives described herein can be used alone and simply mixed with essential detergent ingredient, most notably surfactant, they can also be desirably combined into three-part formulations which combine (a) a non-fragranced detergent base comprising one or more synthetic detergents and (b) one or more of the derivatives described herein. In one embodiment, both aldehydes and acetals are present, such that the aldehydes provide desirable in-package and in-use (wash-time) fragrance, while the acetals provide a long-term fragrance to the laundered textile fabrics.

In formulating the present detergents, the fully-formulated fragrance can be prepared using numerous known odorant ingredients of natural or synthetic origin. The range of the natural raw substances can embrace not only readily-volatile, but also moderately-volatile and slightly-volatile components and that of the synthetics can include representatives from practically all classes of fragrant substances, as will be evident from the following illustrative compilation: natural products, such as tree moss absolute, basil oil, citrus fruit oils (such as bergamot oil, mandarin oil, etc.), mastix absolute, myrtle oil, palmarosa oil, patchouli oil, petitgrain oil Paraguay, wormwood oil, alcohols, such as farnesol, geraniol, linalool, nerol, phenylethyl alcohol, rhodinol, cinnamic alcohol, aldehydes, such as citral, Helional.TM., alpha-hexyl-cinnamaldehyde, hydroxycitronellal, Lilial.TM. (p-tert.butyl-alpha-methyldihydrocinnamaldehyde), methylaonylacetaldehyde, ketones, such as allylionone, alpha-ionone, beta-ionone, isoraldein (isomethyl-alpha-ionone), methylionone, esters, such as allyl phenoxyacetate, benzyl salicylate, cinnamyl propionate, citronellyl acetate, citronellyl ethoxolate, decyl acetate, dimethylbenzylcarbinyl acetate, dimethylbenzylcarbinyl butyrate, ethyl acetoacetate, ethyl acetylacetate, hexenyl isobutyrate, linalyl acetate, methyl dihydrojasmonate, styrallyl acetate, vetiveryl acetate, etc., lactones, such as gamma-undecalactone, various components often used in perfumery, such as musk ketone, indole, p-menthane-8-thiol-3-one, and methyl-eugenol. Likewise, any conventional fragrant acetal or ketal known in the art can be added to the present composition as an optional component of the conventionally formulated perfume (c). Such conventional fragrant acetals and ketals include the well-known methyl and ethyl acetals and ketals, as well as acetals or ketals based on benzaldehyde, those comprising phenylethyl moieties, or more recently developed specialties such as those described in a United States Patent entitled "Acetals and Ketals of Oxo-Tetralins and Oxo-Indanes, see U.S. Pat. No. 5,084,440, issued Jan. 28, 1992, assigned to Givaudan Corp. Of course, other recent synthetic specialties can be included in the perfume compositions for fully-formulated detergents. These include the enol ethers of alkylsubstituted oxo-tetralins and oxo-indanes as described in U.S. Pat. No. 5,332,725, Jul. 26, 1994, assigned to Givaudan; or Schiff Bases as described in U.S. Pat. No. 5,264,615, Dec. 9, 1991, assigned to Givaudan. It is preferred that the pro-fragrant material be added separately from the conventional fragrances to the detergent compositions of the invention.

### Formulation with other Special-Purpose Fragrance Delivering Compounds

Detergents including the derivatives described herein may further, optionally, if desired, contain other known compounds having the capability to enhance substantivity of a fragrance. Such compounds include, but are not limited to, the aluminium alkoxides such as isobutylaluminium diferanylate as disclosed in U.S. Pat. No. 4,055,634; or the known titanate and zirconate esters or oligoesters of fragrant materials such as those disclosed in U.S. Pat. No. 3,947,574, and U.S. Pat. No. 3,779,932, the contents of each of which are hereby incorporated by reference in their entirey. When using such organoaluminum, organotitanium or organozinc derivatives, they may be incorporated into the present formulations at their art-known levels.

### Beverage Compositions

The improved flavorings described .herein can be incorporated into beverages and impart various flavorings to the beverages. The preferred flavor is lemon, but additional flavors include rose, cinnamon, lime, and the like. The beverage composition can be a cola beverage composition, and can also be coffee, tea, dairy beverage, fruit juice drink, orange drink, lemon-lime drink, beer, malt beverages, or other flavored beverage. The beverages can be in liquid or powdered form.

The beverage compositions can also include one or more flavoring agents; artificial colorants; vitamin additives; preservatives; caffeine additives; water; acidulants; thickeners; buffering agents; emulsifiers; and or fruit juice concentrates.

Artificial colorants which may be used include caramel color, yellow 6 and yellow 5. Useful vitamin additives include vitamin B2, vitamin B6, vitamin B12, vitamin C (ascorbic acid), niacin, pantothenic acid, biotin and folic acid. Suitable preservatives include sodium or potassium benzoate. Salts which may be used include sodium, potassium and magnesium chloride. Exemplary emulsifiers are gum arabic and purity gum, and a useful thickener is pectin. Suitable acidulants include citric, phosphoric and malic acid, and potential buffering agents include sodium and potassium citrate.

In one embodiment, the beverage is a carbonated cola beverage. The pH is generally about 2.8 and the following ingredients can be used to make the syrup for these compositions: Flavor Concentrate, including one or more of the derivatives described herein (22.22 ml), 80% Phosphoric Acid (5.55 g), Citric Acid (0.267 g), Caffeine (1.24 g), artificial sweetener, sugar or corn syrup (to taste, depending on the actual sweetener) and Potassium Citrate (4.07 g). The beverage composition can be prepared, for example, by mixing the foregoing syrup with carbonated water in a proportion of 50 ml syrup to 250 ml of carbonated water.

In another embodiment, the beverage is a beer or malt beverage. Preferred flavorings for beer and malt beverages include lemon, lime and lemon-lime. Advantageously, the flavorings include citral derivatives in which one of both of the double bonds are replaced with a cyclopropane group, where the cyclopropane groups can, independently, be unsubstituted, or include one or two alkyl or substituted alkyl groups, preferably methyl groups. The amount of flavoring can be adjusted according to taste.

### Orally-Delivered Products

Flavored food and pharmaceutical compositions including one or more of the derivatives described herein can also be prepared. The derivatives can be incorporated into conventional foodstuffs using techniques well known to those of skill in the art. Alternatively, the derivatives can be incorporated within polymeric particles, which can, in turn, be dispersed within and/or over a surface of an orally-deliverable matrix material, which is usually a solid or semi-solid substrate. When used in chewable compositions, the derivatives can be released into the orally-deliverable polymeric matrix material as the composition is chewed and held in the mouth, thus prolonging the flavor of the composition. In the case of dried powders and mixes, the flavor can be made available as the product is consumed or be released into the matrix material as the composition is further processed. When two flavors are combined with the polymeric particles, the relative amounts of the additives can be selected to provide simultaneous release and exhaustion of the compounds.

In one embodiment, the flavored composition includes an orally-deliverable matrix material; a plurality of water insoluble polymeric particles dispersed in the orally-deliverable matrix material, where the polymeric particles individually define networks of internal pores and are non-degradable in the digestive tract; and one or more derivatives as described herein entrapped within the internal pore networks. The derivatives are released as the matrix is chewed, dissolved in the mouth, or undergoes further processing selected from the group consisting of liquid addition, dry blending, stirring, mixing, heating, baking, and cooking. The orally-deliverable matrix material can be selected from the group consisting of gums, latex materials, crystallized sugars, amorphous sugars, fondants, nougats, jams, jellies, pastes, powders, dry blends, dehydrated food mixes, baked goods, batters, doughs, tablets, and lozenges.

### Chewing Gum

A flavorless gum base can be combined with a citral or other suitable derivative as described herein to a desired flavor concentration. Typically, a blade mixer is heated to about 110F, the gum base is preheated so that it is softened, and the gum base is then added to the mixer and allowed to mix for approximately 30 seconds. The flavored derivative is then added to the mixer and mixed for a suitable amount of time. The gum can be then removed from the mixer and rolled to stick thickness on waxed paper while warm.

### Time Release Formulations

In one embodiment, the derivatives described herein are incorporated into a system which can release a fragrance in a controlled manner. These include substrates such as air fresheners, laundry detergents, fabric softeners, deodorants, lotions, and other household items. The fragrances are generally one or more derivatives of aromachemicals as described herein, each present in different quantities. US Pat. No. 4,587,129, the contents of which are hereby incorporated by reference in their entirety, describes a method for preparing gel articles which contain up to 90% by weight of fragrance or perfume oils. The gels are prepared from a polymer having a hydroxy (lower alkoxy) 2-alkeneoate, a hydroxy (lower alkoxy) lower alkyl 2-alkeneoate, or a hydroxy poly (lower alkoxy) lower alkyl 2-alkenoate and polyethylenically unsaturated crosslinking agent. These materials have continuous slow release properties, i.e., they release the fragrance component continuously over a long period of time. Advantageously, all or a portion of those derivatives that include an aldehyde group can be modified to include an acetal group, which can cause the formulations to release fragrance over a period of time as the acetal hydrolyzes to form the aldehyde compound.

Certain aspects of the invention are described in the following paragraphs.

The present invention provides derivatives of aromachemicals comprising at least one double bond, wherein the derivatives replace at least one double bond in the aromachemicals with a three-membered ring, wherein the three membered ring includes the two carbons from which the double bond was derived, each of which is bonded to an oxygen, a sulfur or a C(R)₂ group,
wherein R is, independently, H, C₁₋₅ alkyl or C₁₋₅ substituted alkyl, and
wherein the substituents on the substituted alkyl groups are selected from the group consisting of halo, hydroxy, thiol, thioether, amine, carboxylic acid, ester, nitro, cyano, sulfonic acid, urea, and thiourea.

Preferably at least one R is methyl. When the aromachemical comprises at least one aldehyde group, the derivatives may further replace at least one aldehyde group in the aromachemicals with a nitrile, methyl ether, ester or acetal group.

The invention provides derivatives of aromachemicals comprising at least one double bond, wherein the derivatives replace at least one double bond in the aromachemicals with a thioether linkage.

The invention also provides derivatives of aromachemicals comprising at least one phenyl ring, wherein the derivatives replace at least one phenyl ring in the aromachemicals with a thiophene ring.

The present invention provides a composition comprising a compound as defined above, together with other perfuming ingredients, solvents, or adjuvants of current use in the art of perfumery. The compound is preferably present in an amount of at least 30 percent by weight, more preferably in an amount of at least 60 percent by weight of the compound.

The present invention also provides a perfuming composition or perfumed article containing as a perfuming ingredient a compound, or a mixture of compounds as defined above. The compound or mixture of compounds may be present in admixture with other perfuming ingredients, solvents, or adjuvants of current use in the art.

The perfumed article may be in the form of a perfume or cologne, a soap, a bath or shower gel, a shampoo or other hair care product, a cosmetic preparation, a body deodorant or antiperspirant, an air freshener, a fabric detergent or softener or an all purpose household cleaner.

The invention provides a body deodorant or antiperspirant, containing as a perfuming ingredient a compound, or a mixture of compounds as defined above. The compound or mixture of compounds may be present in admixture with other perfuming ingredients, solvents, or adjuvants of current use in the art.

The invention provides a detergent containing as a perfuming ingredient a compound, or a mixture of compounds of as defined above. The compound or mixture of compounds may be present in admixture with other perfuming ingredients, solvents, or adjuvants of current use in the art.

The invention provides a bleach composition comprising a citral derivative as defined above.

The invention provides a beverage comprising a citral derivative as defined above. The beverage may preferably be selected from the group consisting of beer, malt liquour, lemonade and cola.

The invention provides a flavoured orally-delivered product comprising a citral derivative as defined above.

The invention provides a method to improve, enhance, or modify the odor of a perfuming composition or a perfumed article comprising adding to said composition or said article an effective amount of a compound or a mixture of compounds as defined above. The compound or mixture of compounds is preferably present in admixture with other perfuming ingredients, solvents, or adjuvants of current use in the art. The compounds are preferably present in an amount of at least 30 percent by weight.

The present invention provides compositions, wherein the essential oil is selected from the group consisting of angelica, aleprine, alpha, beta-apocitronellal, bergamotene, pyroterebine, campholene, citronellal, citral, chrysantheme, cyclocitral, cyclolavandulal, faranal, farnesal, isolauranal, ikema, myrthenal, phellandrene, safranal oxime and sorbinal oxime.

The present invention provides compositions wherein, the essential oil is selected from the group consisting of amyl cinnamal, amyl cinnamyl alcohol, cinnamyl alcohol, cinnamal, citral, coumarin, eugenol, geraniol, hydroxycitronellal, lyral, isoeugenol, benzoyl cinnamate, citronellol, farnesol, hexyl cinnamaldehyde, lilial, d-limonene, linalool, demascanone, and gamma-methylionone.

The present invention provides compositions wherein, the essential oil is selected from the group consisting of anethole, anise oil, caraway oil, cardamom oil, carvone, coriander oil, eriodictyon, ethyl vanillin, fennel oil, glycyrrhiza, lavender oil, lemon oil, menthol, nutmeg oil, orange flower oil, peppermint, rosemary oil, rose oil, spearmint oil, thyme oil, tolu balsam and vanillin.

The present invention provides compositions wherein the essential oil is selected from the group consisting of lilial, cyclamenaldehyde, bourgeonal and citral.

Having hereby disclosed the subject matter of the present invention, it should be apparent that many modifications, substitutions, and variations of the present invention are possible in light thereof. It is to be understood that the present invention can be practiced other an as specifically described. Such modifications, substitutions and variations are intended to be within the scope of the present invention.

## Claims

1. A derivative of an aromachemical obtainable by a process which comprises replacing at least one double bond in an aromachemical by a three-membered ring, wherein the three membered ring includes the two carbons of the double bond, each of which is bonded to an oxygen, a sulfur or a C(R)₂ group,
wherein R is, independently, H, C₁₋₅ alkyl or C₁₋₅ substituted alkyl, and
wherein the substituents on the substituted alkyl groups are selected from the group consisting of halo, hydroxy, thiol, thioether, amine, carboxylic acid, ester, nitro, cyano, sulfonic acid, urea, and thiourea.

2. A derivative according to claim 1, wherein the aromachemical is selected from geraniol, nerol, geranonitrile, angelica, aleprine, alpha,beta-apocitronellal, bergamotene, pyroterebine, campholene, citronellal, citral, chrysantheme, cyclocitral, cyclolavandulal, faranal, farnesal, isolauranal, ikema, myrthenal, phellandrene, safranal oxime, sorbinal oxime, phellandreneandrine oxime, citral oxime, amyl cinnamal, amyl cinnamyl alcohol, cinnamyl alcohol, cinnamal, citral, coumarin, eugenol, hydroxycitronellal, lyral, isoeugenol, benzoyl cinnamate, citronellol, farnesol, hexyl cinnamaldehyde, d-limonene, demascanone, damascones (such as alpha damascone), gamma-methylionone, alpha-ionone, beta-ionone, gamma-methyl ionone, irone alpha, methyl dihydrojasmonate, cis-jasmone, methyl amyl ketone, carvone, methyl beta-napthyl ketone, cassione, menthone, cis and trans-4-methyl-2-phenyl-2-pentenal, anethole, anise oil, caraway oil, cardamom oil, carvone, coriander oil, eriodictyon, ethyl vanillin, fennel oil, glycyrrhiza, lavender oil, lemon oil, menthol, nutmeg oil, orange flower oil, peppermint, rosemary oil, rose oil, spearmint oil, thyme oil, tolu balsam, and vanillin.

3. A derivative according to claim 1 obtainable by a process which additionally comprises the replacement of an aldehyde group in the aromachemical with a methyl ether, a nitrile or an acetal group.

4. A derivative according to any one of the preceding claims obtainable from an aromachemical selected from geraniol, nerol, geranonitrile and citral oxime.

5. A derivative according to any one of the preceding claims of formula 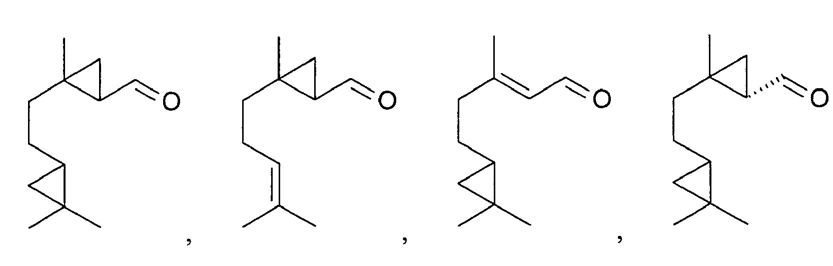 or

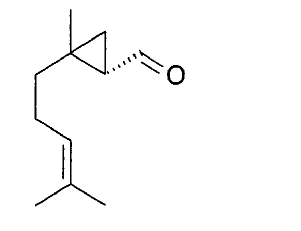

6. A derivative according to any one of claims 1 to 4 in which the three-membered ring contains the group C(R)₂.

7. A derivative according to any one of claims 1 to 4 in which both groups R are H, one group R is methyl and the other is H or both groups R are methyl.

8. A derivative of an aromachemical obtainable by a process which comprises replacing at least one phenyl ring, in an aromachemical with a thiophene ring.

9. A derivative according to claim 8 obtainable from lilial, cyclomenaldehyde, or bourgeonal.

10. A composition comprising a derivative according to any one of the preceding claims, together with other perfuming ingredients, solvents, or adjuvants of current use in the art of perfumery.

11. A perfuming composition or perfumed article containing as a perfuming ingredient a derivative, or a mixture of derivatives, of any of claims 1 to 9.

12. The perfuming composition of claim 11, wherein the derivative or mixture of derivatives is present in admixture with other perfuming ingredients, solvents, or adjuvants of current use in the art.

13. A perfumed article according to claim 11, in the form of a perfume or cologne, a soap, a bath or shower gel, a shampoo or other hair care product, a cosmetic preparation, a body deodorant or antiperspirant, an air freshener, a fabric detergent or softener or an all-purpose household cleaner, optionally comprising other perfuming ingredients, solvents, or adjuvants of current use in the art.

14. A bleach, beverage or flavoured orally-delivered product composition comprising a citral derivative according to any one of claims 1 to 9.

15. A beverage according to claim 14, wherein the beverage is selected from the group consisting of beer, malt liquor, lemonade and cola.

16. The use of a derivative or mixture of derivatives according to any one of claims 1 to 9 as a flavouring and/or fragrance.

17. A method to improve, enhance, or modify the odor of a perfuming composition or a perfumed article comprising adding to said composition or said article an effective amount of a derivative or a mixture of derivatives according to any one of claims 1 to 9.
